# FASCICULE DE BREVET EUROPEEN

(11) **EP 2 178 901 B1**
(45) Date de publication et mention de la délivrance du brevet: **10.05.2017**
(21) Numéro de dépôt: 08827918.7
(22) Date de dépôt: 13.08.2008
(51) Int. Cl.: C07K 1/22, G01N 33/68, C12N 15/11

(54) **PROCEDE DE PURIFICATION OU DE DETECTION D'UNE PROTEINE CIBLE**
VERFAHREN ZUR REINIGUNG ODER ZUM NACHWEIS EINES ZIELPROTEINS
METHOD FOR PURIFYING OR DETECTING A TARGET PROTEIN

(30) Priorité: 14.08.2007 FR 0757072
(43) Date de publication de la demande: 28.04.2010
(60) Demande divisionnaire: 12174841.2
(73) Titulaire: Laboratoire Français du Fractionnement et des Biotechnologies, 91940 Les Ulis (FR)
(72) Inventeur: SIRET, Laurent, F-91700 Villiers Sur Orge (FR); CHTOUROU, Abdessatar, F-78990 Elancourt (FR); DHAINAUT, Frédéric, F-91870 Boissy Le Sec (FR); PERRET, Gérald, F-94600 Choisy Le Roi (FR)
(74) Mandataire: Verhaeghe Bect, Elodie
(86) Numéro de dépôt international: PCT/FR2008/051495
(87) Numéro de publication internationale: WO 2009/024726

(56) Documents cités:
- EP-A2- 1 739 170
- WO-A-02/26932
- WO-A-2005/024042
- WO-A-2007/004748
- WO-A1-93/22337
- LIU XUEMEI ET AL: "RNA aptamers specific for bovine thrombin." JOURNAL OF MOLECULAR RECOGNITION : JMR 2003 JAN-FEB, vol. 16, no. 1, janvier 2003 (2003-01), pages 23-27, XP002479553 ISSN: 0952-3499
- SURUGIU-WARNMARK I ET AL: "Selection of DNA aptamers against rat liver X receptors" BIOCHEMICAL AND BIOPHYSICAL RESEARCH COMMUNICATIONS, ACADEMIC PRESS INC. ORLANDO, FL, US, vol. 332, no. 2, 1 juillet 2005 (2005-07-01), pages 512-517, XP004902506 ISSN: 0006-291X
- LEE S K ET AL: "An RNA aptamer that binds to the beta-catenin interaction domain of TCF-1 protein" BIOCHEMICAL AND BIOPHYSICAL RESEARCH COMMUNICATIONS, ACADEMIC PRESS INC. ORLANDO, FL, US, vol. 327, no. 1, 4 février 2005 (2005-02-04), pages 294-299, XP004697926 ISSN: 0006-291X
- Vivek Babu Kandimalla ET AL: "New Horizons with A Multi Dimensional Tool for Applications in Analytical Chemistry-Aptamer", ANALYTICAL LETTERS, vol. 37, no. 11, 27 December 2004 (2004-12-27), pages 2215-2233, XP055260654, US ISSN: 0003-2719, DOI: 10.1081/AL-200028005

### SEQUENCE LISTING

<110> LFB-BIOTECHNOLOGIES
<120> Procédé de purification ou de détection d'une protéine cible
<130> V361FR
<160> 1
<170> PatentIn version 3.1
<210> 1
   <211> 68
   <212> RNA
   <213> sequence artificielle
<220>
   <223> Aptamere
<400> 1
   gggagagagg aagagggaug ggagccuaug uaacagaugc agaucccuag ucgucccaac      60
   accauaac                                                               68

## Description

### DOMAINE DE L'INVENTION

La présente invention se rapporte au domaine de la purification selon la revendication 1. L'invention est en particulier relative à l'utilisation d'un aptamère pour la purification ou la détection spécifique de protéines d'intérêt dans des milieux, en particulier des solutions, susceptibles de comprendre également des protéines homologues aux protéines d'intérêt.

### ART ANTERIEUR

Les procédés de purification et de détection d'une protéine d'intérêt, qui peut être aussi désignée « protéine cible », comprennent généralement une étape de mise en contact du milieu à tester, susceptible de contenir la protéine d'intérêt, avec des composés ayant la capacité de se lier à ladite protéine d'intérêt. Ces composés capables de se lier à une protéine cible peuvent être de différentes natures. Il peut s'agir (i) de composés organiques comprenant des groupements chimiques possédant des propriétés de liaison à des protéines (tels que DEAE, ammonium quaternaire, chaîne alkyle, silanol), (ii) de protéines (tels que des anticorps), de glycosaminoglycanes (héparine), de colorants (cibacron blue F3GA) ou d'acides nucléiques (tels que des aptamères).

L'efficacité d'un procédé de purification ou de détection d'une protéine cible est liée notamment à l'affinité et à la spécificité du composé qui se lie à la protéine cible.

Avec certains milieux de départ de composition complexe, notamment avec des solutions de départ comprenant de nombreuses protéines distinctes, une protéine cible d'intérêt demeure difficile à purifier et à détecter. C'est le cas notamment avec les milieux complexes de départ dans lesquels a protéine cible à purifier ou à détecter coexiste avec une ou plusieurs protéines distinctes mais fortement homologues. En effet, lorsque la protéine cible se trouve en solution avec une ou plusieurs protéines homologues à cette protéine cible, il devient alors difficile de mettre au point des outils de purification ou de détection permettant de réaliser un haut niveau de discrimination entre la protéine cible d'intérêt et la ou les protéine(s) homologue(s) indésirables, en utilisant des méthodes classiques de purification ou de détection. Même les anticorps, connus pour leur grande spécificité vis-à-vis de protéines cibles, réagissent souvent de façon croisée (« cross reactivity ») également avec plusieurs protéines présentant une homologie de structure avec les protéines cibles.

La difficulté de purifier ou de détecter spécifiquement des protéines présentant une homologie entre elles représente un inconvénient dans de nombreux domaines d'application, que ce soit dans le domaine de la recherche académique visant à étudier spécifiquement une protéine cible indépendamment d'une éventuelle protéine homologue de la protéine cible, ou bien encore dans l'industrie, par exemple pour la mise au point de nouveaux outils de détection ou de purification plus efficaces et, le cas échéant, ayant une meilleure compatibilité avec les exigences administratives ou règlementaires, que les outils de purification ou de détection connus.

La difficulté de purifier ou détecter spécifiquement des protéines présentant une homologie entre elles se rencontre lorsqu'une protéine (ici appelée protéine transgénique) est produite sous une forme recombinante dans un organisme ou un microorganisme transgénique qui exprime aussi naturellement une protéine homologue de ladite protéine transgénique. Sont visées notamment les protéines recombinantes produites dans des organismes possédant de manière naturelle dans leur génome un gène dit « orthologue » codant une protéine fortement homologue à la protéine recombinante codée par un transgène.

Il est courant qu'une protéine transgénique humaine ou animale exprimée dans un animal transgénique soit l'homologue d'une protéine endogène exprimée naturellement chez l'animal transgénique. La production de la protéine naturelle endogène homologue représente un inconvénient technique important dans des situations où l'on cherche à éviter la réalisation d'une co-extraction ou d'une co-détection de la protéine transgénique et de la protéine naturelle endogène homologue.

Lorsque la protéine recombinante transgénique consiste en une protéine d'intérêt thérapeutique, destinée à la fabrication d'un médicament, la présence, dans la préparation purifiée de la protéine recombinante, de toute protéine endogène homologue de la protéine transgénique peut entraîner des effets indésirables pour le patient à qui le médicament est administré, y compris l'induction d'une réponse immunitaire indésirable à l'encontre de la protéine naturelle contaminante, qui est susceptible de réduire l'efficacité du traitement médical et même parfois provoquer des réponses auto-immunes de nature à mettre en danger la vie du patient. De tels problèmes sont rencontrés de plus en plus souvent avec le recours croissant à la fabrication de protéines transgéniques thérapeutiques chez des animaux transgéniques. Afin de proposer des produits thérapeutiques ayant une haute innocuité, les protéines transgéniques doivent donc être purifiées spécifiquement, en présence de très faibles quantités de protéines homologues indésirables, et si possible en l'absence totale de protéines homologues indésirables. De même, les méthodes de détection de protéines cibles d'intérêt doivent être spécifiques, sensibles et performantes.

Les aptamères de type ARN, molécules d'acide ribonucléique monobrin, sélectionnés selon un procédé connu sous le nom de SELEX, sont capables de lier spécifiquement des protéines d'intérêt. Dans la publication Liu et al. RNA aptamers specific for bovine thrombin, J. Mol. Recognit., 2003 :16, 23-27, l'auteur présente un aptamère de type ARN qui se lie à la thrombine bovine mais qui ne se lie pas à la thrombine humaine. Cet article, bien qu'isolé, montre qu'il est possible de sélectionner par le procédé SELEX des aptamères ARN de haute spécificité capables de discriminer des protéines homologues en solution.

Néanmoins à ce jour aucun procédé industriel utilisant des aptamères de haute spécificité servant à discriminer des protéines homologues n'a encore été décrit dans la littérature.

L'utilisation des aptamères de haute spécificité se heurte à de nombreux problèmes techniques. Tout d'abord, les aptamères sont sélectionnés en solution selon le procédé SELEX. Pour être utilisé dans un procédé de purification ou de détection, l'aptamère doit être immobilisé sur un support solide. Or de nombreuses publications montrent que les propriétés de liaison de l'aptamère (affinité, spécificité...) sont altérées lorsque l'aptamère est immobilisé. De plus, les aptamères de type ARN, seul type d'aptamère de haute spécificité décrit dans l'art antérieur, sont des molécules très labiles qui se dégradent à température ambiante. Les aptamères ARN ne sont donc pas compatibles avec les procédés industriels de purification ou de détection. Les ARN chimiquement modifiés pourraient constituer une alternative mais le coût de synthèse actuel pour de telles molécules est totalement incompatible avec une utilisation industrielle.

En raison de leur grande affinité et de leur spécificité relativement élevée pour des protéines d'intérêt, les anticorps sont largement utilisés dans les procédés de purification ou de détection des protéines.

Cependant l'utilisation des anticorps n'est pas toujours bien adaptée à la purification ou à la détection d'une protéine cible. En effet les anticorps sont fabriqués dans des systèmes biologiques avec tous les inconvénients que cela comporte, y compris les risques de contamination par des agents pathogènes, ou encore la difficulté de purifier les anticorps fabriqués. De plus, les anticorps sont généralement immunogènes et peuvent induire de fortes réactions immunitaires s'ils sont administrés chez un patient. Ainsi, lorsqu'un anticorps est utilisé pour purifier une protéine thérapeutique, il y a un risque de retrouver des anticorps ou des fragments d'anticorps dans la solution contenant la protéine thérapeutique et donc d'engendrer des effets indésirables chez le patient auquel on administre une telle solution. C'est pourquoi l'utilisation des anticorps dans des procédés de purification est devenue difficilement compatible avec certaines exigences administratives, en particulier dans les domaines agro-alimentaire et pharmaceutique. On peut ajouter qu'avec les anticorps, les moyens de sanitisation sont limités, du fait de leur nature protéique fragile, notamment lorsque sont utilisées (i) des conditions dénaturantes (par exemple en présence d'urée, de DMSO, etc.), (ii) des pH très acides ou très basique, (iii) certains solvants organiques délétères, ou bien encore (iv) des températures élevées. En dernier lieu, on rappelle que la sélection d'un anticorps est longue (6 mois environ) et la production d'un anticorps purifié est complexe à mettre en oeuvre, ce qui entraîne des coûts de sélection et de fabrication relativement élevés.

Il existe donc un besoin pour la mise au point de nouveaux outils de purification ou de détection de protéines d'intérêt, qui soient efficaces, qui soient plus simples à mettre en oeuvre et qui soient plus sûrs que les procédés connus. De tels procédés améliorés doivent permettre la purification ou la détection sélective d'une protéine cible présente dans une solution lorsque ladite solution est susceptible de contenir aussi une protéine homologue à la protéine cible. Plus particulièrement, il existe un besoin pour la mise au point de nouveaux outils de purification ou de détection permettant d'améliorer les conditions de sécurité et d'innocuité pour l'homme des produits issus la production des protéines transgéniques thérapeutiques à partir d'organismes transgéniques.

### RESUME DE L'INVENTION

La présente invention a pour objet un procédé pour la purification selon la revendication 1 d'une protéine cible présente dans une solution, ledit procédé comprenant, préalablement à la réalisation de l'étape de purification, une étape de mise en contact de ladite solution avec un aptamère ADN immobilisé sur un support solide via un espaceur, ledit aptamère immobilisé se liant spécifiquement à ladite protéine cible mais ne se liant pas à une protéine homologue à ladite protéine cible susceptible d'être également présente en solution.

La présente invention concerne donc aussi l'utilisation d'un aptamère ADN immobilisé sur un support solide via un espaceur, ledit aptamère immobilisé se liant spécifiquement à une protéine cible, pour la purification ou la détection de ladite protéine cible présente dans une solution, caractérisée en ce que ledit aptamère immobilisé ne se lie pas à une protéine homologue de ladite protéine cible et que ladite solution est susceptible de contenir au moins une protéine homologue de ladite protéine cible.

La présente invention concerne également un procédé pour purifier spécifiquement une protéine cible à partir d'une solution qui contient ladite protéine cible et qui est susceptible de contenir une protéine homologue de la protéine cible comprenant les étapes de (i) fournir un support solide comprenant un aptamère ADN immobilisé audit support solide via un espaceur caractérisé en ce que ledit aptamère immobilisé se lie spécifiquement à ladite protéine cible mais ne se lie pas à une protéine homologue de la protéine cible, (ii) mettre en contact ledit support solide avec ladite solution, et (iii) récupérer la protéine cible liée audit aptamère.

### DESCRIPTION DES FIGURES

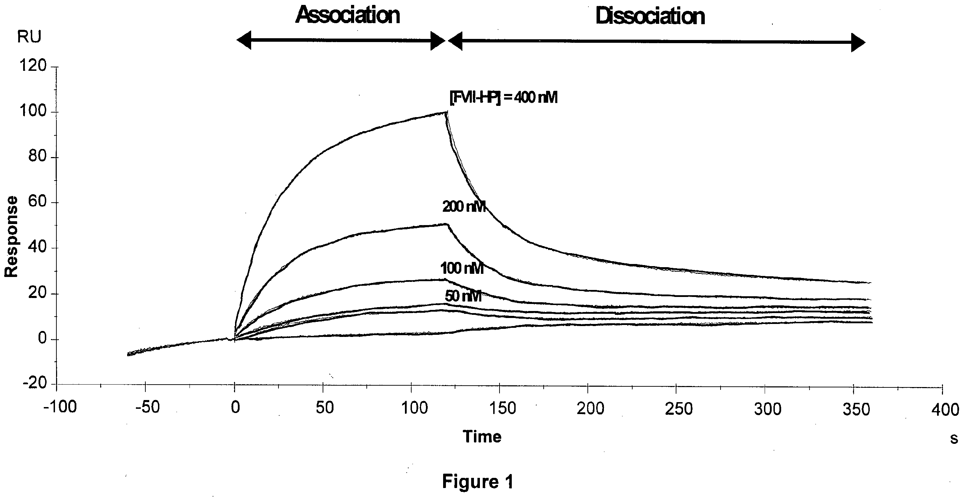

La **figure 1** illustre la liaison entre un aptamère qui se lie spécifiquement au FVII humain et le FVII humain plasmatique (FVII HP). La figure 1 représente un graphique représentant la liaison en fonction du temps de l'aptamère anti-FVII humain et du FVII HP à des concentrations en FVII HP comprises entre 50 à 400 nM.

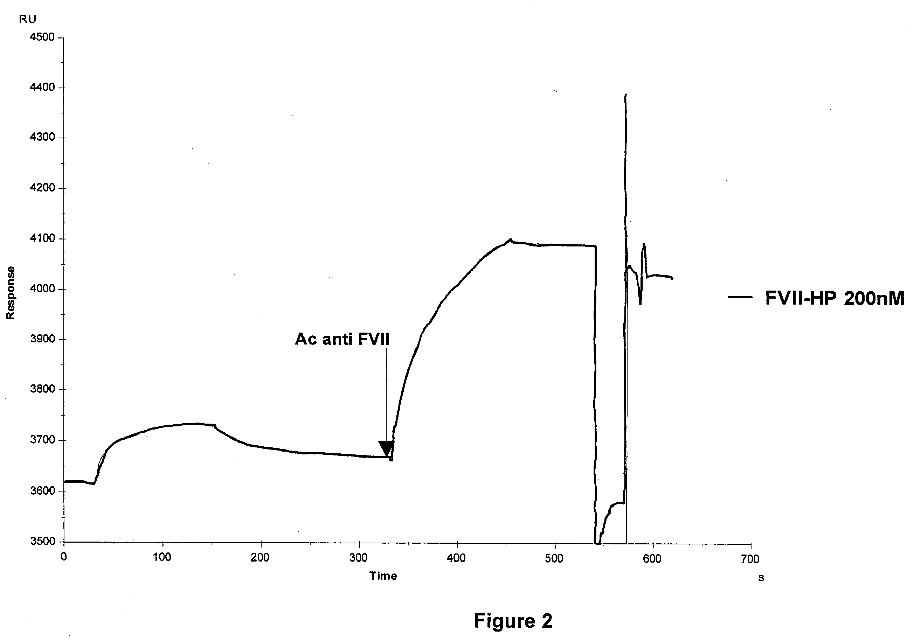

La **figure 2** illustre la spécificité de l'interaction entre un aptamère immobilisé sur une puce et le FVII HP. La figure 2 représente un graphique représentant la liaison en fonction du temps de l'aptamère qui se lie spécifiquement au FVII humain immobilisé sur une puce et du FVII HP en présence ou non d'un anticorps polyclonal anti-FVII.

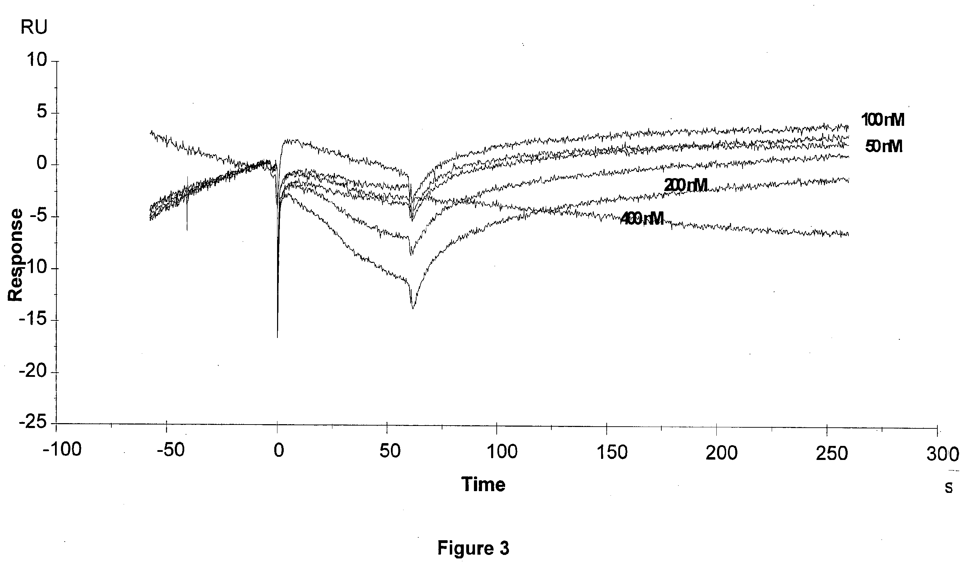

La **figure 3** illustre l'absence de liaison entre l'aptamère qui se lie spécifiquement au FVII humain et le FVII de lapin. La figure 3 représente un graphique représentant la liaison en fonction du temps de l'aptamère qui se lie spécifiquement au FVII humain et le FVII de lapin à des concentrations en FVII de lapin comprises entre 50 et 400 nM.

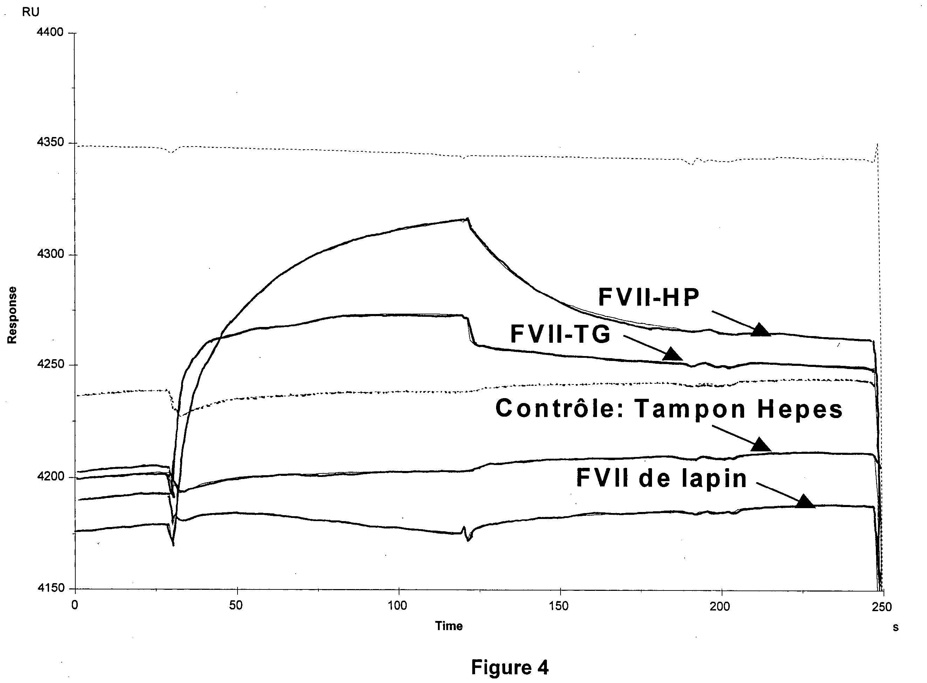

La **figure 4** illustre les différences d'affinité entre l'aptamère qui se lie spécifiquement au FVII humain et le FVII HP, le FVII de lapin ou le FVII humain transgénique. La figure 4 représente un graphique représentant la liaison en fonction du temps de l'aptamère qui se lie spécifiquement au FVII humain et le FVII HP, le FVII de lapin ou le FVII humain transgénique à une concentration de 400nM pour chaque FVII.

### DESCRIPTION DE L'INVENTION

La présente invention fournit un procédé pour la purification d'une protéine cible présente dans une solution, ledit procédé comprenant, préalablement à la réalisation de l'étape de purification, une étape de mise en contact de ladite solution avec un aptamère ADN immobilisé sur un support solide via un espaceur, ledit aptamère immobilisé se liant spécifiquement à ladite protéine cible mais ne se liant pas à une protéine homologue à ladite protéine cible susceptible d'être également présente en solution.

L'invention concerne aussi l'utilisation d'un ADN immobilisé sur un support solide via un espaceur, ledit aptamère immobilisé se liant spécifiquement à une protéine cible, pour la purification ou la détection de ladite protéine cible présente dans une solution, caractérisée en ce que ledit aptamère immobilisé ne se lie pas à une protéine homologue de la protéine cible et que ladite solution est susceptible de contenir au moins une protéine homologue de la protéine cible.

Dans le procédé pour la purification d'une protéine cible d'intérêt, un complexe est sélectivement formé entre (i) ledit aptamère reconnaissant spécifiquement une protéine cible déterminée et (ii) la protéine cible, si celle-ci est contenue dans le milieu de départ, typiquement la solution de départ. Puis, la protéine cible à purifier est récupérée par dissociation du complexe préalablement formé avec ledit aptamère.

Dans le procédé pour la détection d'une protéine cible d'intérêt, un complexe est sélectivement formé entre (i) ledit aptamère reconnaissant spécifiquement une protéine cible déterminée et (ii) la protéine cible, si celle-ci est contenue dans le milieu de départ, typiquement la solution de départ. Puis, sont détectés notamment (i) soit les complexes formés entre ledit aptamère et ladite protéine d'intérêt, (ii) soit la protéine cible d'intérêt, qu'elle soit complexée audit aptamère ou qu'elle soit sous forme libre après dissociation des complexes préalablement formés. L'objet de la présente invention concerne un procédé pour purifier spécifiquement une protéine cible à partir d'une solution qui contient ladite protéine cible et qui est susceptible de contenir une protéine homologue de la protéine cible comprenant les étapes de :
a) fournir un support solide comprenant un aptamère ADN immobilisé audit support solide via un espaceur caractérisé en ce que ledit aptamère immobilisé se lie spécifiquement à ladite protéine cible mais ne se lie pas à une protéine homologue de la protéine cible,
b) mettre en contact ledit support solide avec ladite solution, et
c) récupérer la protéine cible liée audit aptamère.
La présente demande divulgue aussi un procédé pour détecter spécifiquement une protéine cible à partir d'une solution qui contient ladite protéine cible et qui est susceptible de contenir une protéine homologue de la protéine cible comprenant les étapes de :
a) mettre en contact ladite solution avec un aptamère ADN immobilisé sur un support solide via un espaceur ledit aptamère immobilisé se liant spécifiquement à ladite protéine cible mais ne se liant pas à ladite protéine homologue de la protéine cible, et
b) détecter les complexes formés entre ledit aptamère et ladite protéine cible.

Le terme « aptamère » tel qu'utilisé ici désigne une molécule d'acide nucléique monobrin, capable de se lier de manière spécifique à une protéine. Les aptamères comprennent généralement entre 5 et 120 nucléotides et peuvent être sélectionnés in vitro selon un procédé connu sous le nom de SELEX (Systematic Evolution of Ligands by Exponential Enrichment). Les aptamères présentent de nombreux avantages. De par leur nature oligonucléotidique les aptamères possèdent une faible immunogenicité et une résistance importante à des conditions physico-chimiques stringentes (présence d'urée, de DMSO, d'un pH très acides ou très basique, utilisation de solvants organiques ou de température élevée) permettant des stratégies de sanitisation variées dans le cadre d'un usage en tant que ligand d'affinité. De plus leur sélectivité est importante. Enfin la production d'aptamères implique des coûts relativement limités.

Le terme « aptamère ADN » tel qu'utilisé ici désigne un aptamère constitué de désoxyribonucléotides, à la différence des aptamères ARN qui sont constitués de ribonucléotides. Les aptamères ADN présentent l'avantage d'être stables en solution, et donc exploitables industriellement pour la purification ou la détection d'une protéine cible.

Le terme « protéine » tel qu'utilisé ici correspond à un polymère d'acides aminés. Cela comprend les protéines, les fragments de protéine, les protéines modifiées génétiquement, les oligopeptides et leurs analogues. La protéine peut être un anticorps, une protéine antivirale, une hormone, un facteur de croissance, un facteur de la coagulation tel que le facteur VII (FVII), le facteur VIII (FVIII), le facteur X (FX), le facteur IX (Facteur IX), le facteur XI (FXI), le facteur XII (FXII), le facteur XIII (FXIII), le facteur II (thrombine), l'antithrombine III (AT III) le cofacteur II de l'héparine (HCII), la protéine C (PC), la thrombomoduline (TM), la protéine S (PS), le facteur V (FV), le facteur de Willlerbrand (FvW) et l'inhibiteur de la voie du facteur tissulaire (TFPI). De préférence, la protéine est un FVII natif ou modifié, ou un fragment de celui-ci.

Comme déjà indiqué précédemment, on utilise dans les procédés de l'invention un « aptamère ADN immobilisé sur un support solide via un espaceur » (également appelé « aptamère » dans la suite de la description) ayant la capacité à se lier sélectivement à une protéine cible déterminée, ledit aptamère possédant une capacité réduite ou nulle à se lier à des protéines « homologues » à ladite protéine cible déterminée. Cela signifie que l'on utilise selon l'invention exclusivement des aptamères ayant d'excellentes propriétés de discrimination entre la protéine cible d'intérêt et des protéines distinctes de ladite protéine cible, bien que possédant une structure proche de ladite protéine cible.

Dans certains modes de réalisation de l'invention, la capacité de l'aptamère utilisé à discriminer la protéine cible des protéines « homologues » à la protéine cible est telle que ledit aptamère possède une affinité pour la protéine cible d'intérêt définie par une valeur de constante de dissociation (Kd), exprimée en concentration molaire, qui est inférieure d'au moins un ordre de grandeur par rapport à la constante de dissociation dudit aptamère pour la protéine « homologue » la plus proche connue.

Dans certains autres modes de réalisation de l'invention, l'aptamère ne se lie pas à une protéine « homologue » à la protéine cible d'intérêt. Selon l'invention, un aptamère ne se lie pas à une protéine homologue lorsque la liaison de la protéine homologue audit aptamère n'est pas détectable selon les techniques de mesure conventionnelles, par exemple selon la technique de détection et de mesure de fixation de type Biacore®. A titre illustratif, on a montré dans les exemples que l'aptamère de séquence SEQ ID N°1 qui se lie sélectivement au Facteur VII humain ne se lie pas de façon détectable au Facteur VII de lapin, en utilisant la technique de masure de liaison Biacore®.

La « protéine cible » avec laquelle se lie sélectivement l'aptamère peut être tout type de protéine. La protéine cible peut notamment consister en une protéine d'intérêt thérapeutique.

La « protéine homologue » ou « protéine homologue à la protéine cible » consiste en une protéine qui présente une grande homologie structurelle avec la protéine cible.

Le terme « homologie » tel qu'utilisé dans la présente invention correspond à des homologies qui sont essentiellement de deux natures, respectivement (i) une homologie résultant de différences dans la séquence d'acides aminés entre la protéine cible et la protéine homologue et (ii) une homologie résultant de différences dans les groupements chimiques additionnels fixés de manière covalente sur les chaînes latérales des acides aminés, y compris des différences dans les chaînes osidiques. L'homme du métier comprend aisément que la protéine cible d'intérêt et une protéine « homologue » peuvent se distinguer à la fois par des différences dans la séquence d'acides aminés et par des différences dans les chaînes osidiques. A titre illustratif, la combinaison des deux types de différences structurelles est rencontrée lorsqu'une différence dans la séquence d'acides aminés a lieu sur un acide aminé consistant en un site de glycosylation de la protéine cible ou de la protéine homologue.

Selon l'invention, la protéine cible déterminée et une protéine homologue correspondante ont des séquences qui possèdent entre elles au moins 50% d'identité en acides aminés. Selon l'invention, le pourcentage d'identité entre deux séquences en acides aminés, ou entre deux séquences de nucléotides, peut être calculée par Emboss matcher, EBLOSUM62 en utilisant les paramètres par défaut dont Gap_penalty : 10.0 et Extend_penalty : 0.5.

De préférence, l'identité de séquence en acides aminés entre la protéine cible est la protéine homologue est d'au moins 50%, 55%, 60%, 65%, 70%, 75%, 80%, 85%, 90%, 91%, 92%, 93%, 94%, 95%, 96%, 97%, 98%, ou 99%.

Comme indiqué précédemment, la distinction entre la protéine cible d'intérêt et la protéine homologue peut résulter de différences dans la glycosylation de ces deux protéines. Une différence de glycosylation concerne deux protéines ayant la même séquence en acides aminés ou ayant des séquences en acides aminés proches mais dont les structures glycaniques diffèrent. Les structures glycaniques sont des chaînes de sucres greffées aux protéines au niveau de séquences consensus d'acides aminés. Les différences de glycosylation peuvent porter sur les N- ou O-glycosylation ainsi que sur les ramifications glycosidiques qui se forment sur ces N- ou O-glycosylations. La différence de glycosylation peut porter sur la présence ou l'absence de telles chaînes ou ramifications mais aussi sur la présence ou l'absence de certains sucres tels que les résidus fucose, mannose, glucosamine ou galactosamine. La glycosylation des protéines peut être analysée de différentes manières. Une méthode d'analyse de la glycosylation consiste à déglycosyler une protéine avec une ou plusieurs enzymes (PNGaseF, N-glycosidase) ou chimiquement (Hydrasynolyse, β-élimination) puis à analyser directement les glycanes obtenus sur gel, électrophorèse capillaire ou HPLC (avec détection ultra violet, fluorescente ou de spectrométrie de masse) ou analyser après dépolymérisation enzymatique (Neuraminidase et (-galactosidase) ou chimique (Hydrasynolyse, β-élimination) dans le but de séquencer les glycanes. Une autre méthode d'analyse consiste à employer la spectrométrie de masse sur la protéine entière, ces techniques sont connues sous les noms de MALDI (Matrix-Assisted Laser Desorption/lonisation), MALDI-TOF (Time Of Flight), ESI (Electrospray Ionisation).

Dans certains modes de réalisation de l'invention, un aptamère selon l'invention consiste en un aptamère qui se lie spécifiquement à un facteur de la coagulation choisi parmi le facteur VII (FVII), le facteur VIII (FVIII), le facteur X (FX), le facteur IX (Facteur IX), le facteur XI (FXI), le facteur XII (FXII), le facteur XIII (FXIII), le facteur II (THROMBINE), l'antithrombine III (AT III) le cofacteur II de l'héparine (HCII), la protéine C (PC), la thrombomoduline (TM), la protéine S (PS), le facteur V (FV), le facteur de Willlerbrand (FvW) et l'inhibiteur de la voie du facteur tissulaire (TFPI). Avantageusement l'aptamère se lie spécifiquement au FVII. De préférence l'aptamère selon l'invention se lie spécifiquement au FVII humain mais ne se lie pas au FVII de lapin. Inversement un aptamère selon l'invention se lie au FVII de lapin mais ne se lie pas au FVII humain.

Un aptamère qui se lie spécifiquement à un FVII humain mais qui ne se lie pas à un FVII de lapin peut être un aptamère ayant une séquence nucléotidique ayant au moins 80% d'identité en nucléotides avec la séquence SEQ ID n°1.

Avantageusement, l'aptamère de l'invention se lie à la protéine cible avec une affinité caractérisée par une valeur de constante de dissociation (Kd) comprise entre 1 pM et 10 µM, de préférence comprise entre 10 nM et 10 µM. Avantageusement, l'affinité de l'aptamère pour la protéine cible est 1000 à 10 000 fois supérieure à l'affinité de l'aptamère pour la protéine homologue.

Dans le procédé de purification ou de détection selon l'invention, l'aptamère ADN est immobilisé sur un support solide via un espaceur. L'aptamère immobilisé sur un support solide est particulièrement bien adapté pour la détection ou la purification d'une protéine cible présente dans une solution.

Le terme « espaceur » tel qu'utilisé ici désigne une molécule qui est intercalée entre l'aptamère et le support solide. Avantageusement l'espaceur est lié à la fois à une extrémité de l'aptamère et au support solide. Cette construction avec espaceur a pour avantage de ne pas immobiliser directement l'aptamère sur le support solide. La nature de l'espaceur peut être choisie selon les connaissances de l'homme du métier, de préférence l'espaceur est une séquence oligonucléotidique non spécifique ou du Polyéthylène Glycol (PEG). Lorsqu'il s'agit d'une séquence oligonucléotidique non spécifique, ladite séquence comporte de préférence au moins 5 nucléotides, de préférence entre 5 et 15 nucléotides.

Pour immobiliser l'aptamère sur un espaceur, l'aptamère peut être modifié chimiquement avec différents groupements chimiques tels que des groupements permettant d'immobiliser l'aptamère de façon covalente tels que des thiols, des amines ou tout autre groupement capable de réagir avec des groupements chimiques présents sur le support, ou des groupements permettant d'immobiliser l'aptamère de façon non-covalente tels le système biotine-streptavidine. Ces techniques sont également applicables pour immobiliser l'espaceur sur le support solide.

Une fois immobilisé sur le support solide via l'espaceur, l'aptamère est avantageusement modifié à son extrémité libre (extrémité non liée à l'espaceur) grâce à, et sans s'y limiter, un nucléotide chimiquement modifié (tel que 2'omethyl ou 2'fluoropyrimidine, 2' ribopurine, phosphoramidite), un nucléotide inversé ou un groupement chimique (PEG, polycations, cholestérol). Ces modifications permettent de protéger l'aptamère des dégradations enzymatiques.

Le support solide peut être une colonne de chromatographie d'affinité composée d'un gel dérivé de l'agarose, de la cellulose ou d'un gel synthétique tel qu'un dérivé acrylamide, méthacrylate ou polystyrène ; une puce telle qu'une puce adaptée à la résonance plasmonique de surface ; une membrane telle qu'une membrane polyamide, polyacrylonitrile ou polyester ; une bille magnétique ou paramagnétique.

Avantageusement, la solution qui contient la protéine cible et qui est susceptible de contenir une protéine homologue de la protéine cible est une solution biologique tel qu'un fluide corporel, une cellule, un broyat cellulaire, un tissu, un broyat tissulaire, un organe ou un organisme entier. De préférence la solution est une solution biologique liquide provenant d'un animal tel que du sang, un dérivé du sang (dérivé sanguin), du lait de mammifère ou un dérivé du lait de mammifère. Il peut s'agir du plasma, du cryoprécipité du plasma, du lait clarifié ou leurs dérivés. Un animal selon la présente invention est un organisme vivant pluricellulaire, eucaryote, dépourvus de chloroplastes et non humain. Dans un mode de réalisation particulièrement préféré, la solution provient d'un animal transgénique. Avantageusement la solution est du lait de mammifère ou un dérivé du lait de mammifère transgénique. Selon l'invention, un animal transgénique est un mammifère telle qu'une vache, une chèvre, une lapine, un cochon, un singe, un rat ,une souris ou un oiseau ou un insecte tel qu'un moustique, une mouche, un ver à soie. Dans un mode de réalisation préféré, l'animal transgénique est un mammifère transgénique, de préférence une lapine transgénique. Avantageusement, le mammifère transgénique produit une protéine transgénique dans ses glandes mammaires sous le contrôle d'un promoteur spécifique permettant l'expression de ladite protéine transgénique dans le lait dudit mammifère transgénique.

Une méthode de production d'une protéine transgénique dans le lait d'un animal transgénique peut comporter les étapes suivantes : une molécule d'ADN comprenant un gène codant pour la protéine transgénique, ce gène étant sous le contrôle d'un promoteur d'une protéine sécrétée naturellement dans le lait (tels que le promoteur de la caséine, de la béta-caséine, de la lactalbumine, de la béta-lactoglobuline ou le promoteur WAP) est intégrée dans un embryon d'un mammifère non humain. L'embryon est ensuite placé dans une femelle de mammifère de la même espèce. Une fois que le mammifère issu de l'embryon s'est suffisamment développé, la lactation du mammifère est induite, puis le lait collecté. Le lait contient alors ladite protéine transgénique.

Un exemple de procédé de préparation de protéine dans le lait d'une femelle de mammifère autre que l'être humain est donné dans le document EP 0 527 063, dont l'enseignement peut être repris pour la production de la protéine de l'invention. Un plasmide contenant le promoteur WAP (Whey Acidic Protein) est fabriqué par introduction d'une séquence comportant le promoteur du gène WAP, ce plasmide étant réalisé de manière à pouvoir recevoir un gène étranger placé sous la dépendance du promoteur WAP. Le plasmide contenant le promoteur et le gène codant pour la protéine de l'invention sont utilisés pour obtenir des lapines transgéniques, par microinjection dans le pronucléus mâle d'embryons de lapines. Les embryons sont ensuite transférés dans l'oviducte de femelles hormonalement préparées. La présence des transgènes est révélée par la technique de Southern à partir de l'ADN extrait des lapereaux transgéniques obtenus. Les concentrations dans le lait des animaux sont évaluées à l'aide de tests radioimmunologiques spécifiques.

D'autres documents décrivent des procédés de préparation de protéines dans le lait d'une femelle de mammifère autre que l'homme. On peut citer, sans s'y limiter les documents US 7,045,676 (souris transgénique) et EP 1 739 170. (production de facteur von Willebrand dans un mammifère transgénique).

Ainsi, l'invention a aussi pour objet l'utilisation d'un aptamère ADN immobilisé sur un support solide via un espaceur, ledit aptamère immobilisé se liant spécifiquement au FVII humain, pour la purification ou la détection dudit FVII humain présent dans du lait de lapine transgénique, caractérisée en ce que ledit aptamère immobilisé ne se lie pas au FVII de lapin et que ledit lait de lapine transgénique est susceptible de contenir du FVII de lapin.

L'invention a aussi pour objet l'utilisation d'un aptamère ADN immobilisé sur un support solide via un espaceur, ledit aptamère immobilisé se liant spécifiquement au FVII de lapin, pour la détection dudit FVII de lapin susceptible d'être présent dans du lait de lapine transgénique, caractérisée en ce que ledit aptamère immobilisé ne se lie pas au FVII humain et que ledit lait de lapine transgénique contient du FVII humain.

Le FVII humain et le FVII de lapin ont une homologie de séquence en acides aminés d'environ 75% (calculé par BLAST).

Un autre objet de la présente invention concerne un procédé de sélection d'un aptamère qui se lie spécifiquement à une protéine cible mais qui ne se lie pas à une protéine homologue de la protéine cible comprenant les étapes de :
a) mettre en contact un mélange d'aptamères avec ladite protéine homologue de la protéine cible dans des conditions favorables à la liaison, et récupérer les aptamères qui ne se lient pas à la protéine homologue,
b) mettre en contact le mélange d'aptamères obtenu à l'étape a) avec ladite protéine cible dans des conditions favorables à la liaison,
c) séparer les aptamères non liés des aptamères qui ont été liés à ladite protéine cible,
d) dissocier les paires aptamère-protéine cible,
e) amplifier les aptamères dissociés pour donner un mélange enrichi en aptamère qui se lie à la protéine cible mais qui ne se lie pas à la protéine homologue de la protéine cible, et
f) réitérer les étapes a), b), c) d), e) sur autant de cycles qu'on le souhaite pour obtenir le(s)dit(s) aptamère(s) qui se lie(nt) spécifiquement à la molécule cible mais qui ne se lie(nt) pas à une protéine homologue de la protéine cible.

Le procédé de sélection d'un aptamère de l'invention s'apparente au procédé SELEX décrit dans l'art antérieur avec en plus une étape dite de « soustraction » (étape a). Ainsi le procédé de l'invention est appelé « SELEX par soustraction ».

Le procédé SELEX de sélection des aptamères consiste à mettre en présence une protéine avec une librairie combinatoire d'acides nucléiques, ADN ou ARN (en général 10¹⁵ molécules), les acides nucléiques ne se liant pas à la cible sont éliminé, les acides nucléiques se liant à la cible sont isolés et amplifiés. Le procédé est répété jusqu'à ce que la solution soit suffisamment enrichie avec les acides nucléiques ayant une bonne affinité pour la protéine d'intérêt (Tuerk et Gold, « Systematic evolution of ligands by exponential enrichment : RNA ligands to bacteriophage T4 DNA polymerase » (1990) Science, 249(4968) :505-10 et Ellington et Szostak, « In vitro selection of RNA molecules that bind specific ligands », (1990) Nature Aug 30;346(6287):818-22). D'autres exemples de procédé SELEX sont donnés dans les documents EP 0 786 469, EP 668 931, EP 1 695 978, EP 1 493 825 dont les enseignements peuvent être repris dans la réalisation du procédé de sélection d'un aptamère de l'invention.

L'étape de séparation (étape c) peut être réalisée par tout procédé qui permet de séparer les aptamères liés à la protéine cible, appelé paires aptamère-protéine cible, des aptamères non liés à la protéine cible. La séparation peut s'effectuer par différents procédés connus dans l'art antérieur. Ainsi les paires aptamère-protéine cible peuvent être retenues sur des filtres de nitrocellulose tandis que les aptamères libres ne sont pas retenus. Des colonnes qui retiennent spécifiquement les paires aptamère-protéine cible peuvent également être utilisés pour l'étape de séparation. D'autres procédés peuvent également être utilisés comme l'extraction liquide-liquide, la filtration sur gel retard ou la centrifugation sur gradient de densité. Le choix du procédé de séparation va dépendre des propriétés de la protéine cible et de la paire aptamère-protéine cible et peut être fait en fonction de principes connus de l'homme du métier.

L'étape de dissociation (étape d) peut être réalisé par des méthodes permettant de dissocier des entités chimiques. Avantageusement, la dissociation se fait par une augmentation de la force ionique ou une modification du pH.

L'amplification réalisée à l'étape e) peut être réalisée par (tout procédé qui permet d'augmenter la quantité ou le nombre de copies d'un aptamère. Avantageusement, l'amplification d'un aptamère est réalisée par la technique de PCR (Polymerase Chain Réaction). Avantageusement, l'amplification est obtenue soit par PCR s'il s'agit d'ADN soit par RT-PCR s'il s'agit d'ARN.

Le procédé « SELEX par soustraction » est caractérisé par l'étape dite de « soustraction » (étape a) qui permet d'éliminer les aptamères qui se lient à la protéine homologue de la protéine cible. Après avoir réalisé un cycle comportant les étapes a), b), c), d) et e), les cycles suivants peuvent soit reprendre les étapes a), b), c), d) et e) soit partir directement de l'étape b) à partir d'un pool d'aptamères amplifiés à l'étape e) du cycle précédent. Ainsi l'étape a) de soustraction est réalisée au début de chaque nouveau cycle ou alternativement après avoir effectué au moins un cycles sans étape a) et jusqu'à obtenir des aptamères qui se lient spécifiquement à la protéine cible mais qui ne se lient pas à la protéine homologue de la protéine cible. Avantageusement, chaque étape a) est réalisée tous les deux ou trois cycles.

Les exemples ci-dessous permettent d'illustrer l'invention sans en limiter la portée.

### EXEMPLES

### Exemple 1 : Liaison de l'aptamère (SEQ n°1) avec le FVII humain

Un aptamère de séquence SEQ n°1 a été synthétisé par la société Sigma-Proligo.
**SEQ n°1 : 5'PGGGAGAGAG**GAAGAGGGAUGGGAGCCUAUGUAACAGAUGCAGAUCCCUA GUCGUCCCAACACCAUAAC**-3'-Biotine**

Cet aptamère est modifié sur son extrémité 3' par le greffage d'une biotine. Les bases nucléotidique notées en gras (SEQ n°1) sont 2'O-méthylées, ce qui permet de stabiliser l'aptamère en le rendant plus résistant aux nucléases. L'aptamère a ensuite été immobilisé sur une puce SA (Biacore GE) contenant de l'avidine. L'aptamère est ainsi immobilisé de façon orienté par son extrémité 3' avec un taux d'immobilisation de 2700 RU (1 RU correspond approximativement à 1 pg de produit immobilisé par mm²).

Du FVII humain purifié à partir du plasma (FVII HP, pureté : 99%) a été dilué dans du tampon de course (Hepes 20mM pH=8, NaCl 50mM, CaCl₂ 2mM et BSA 0.01 %) de manière à obtenir quatre échantillons de concentration en FVII HP de 50, 100, 200 et 400 nM. Chaque échantillon a été injecté de façon séquentielle sur une même puces contenant l'aptamère immobilisé par une interaction biotine-streptavidine. Des contrôles sont obtenus en injectant des blancs ne contenant que du tampon de course. Toutes les injections ont été réalisées avec un débit 20µl/min pendant 120 sec, après l'injection, du tampon de course a été injecté sur la puce à un débit identique pendant 240 sec. Du tampon d'élution (NaCl, 5M) a ensuite été injecté pendant 60 sec avec un débit de 30µl/min pour décrocher le FVII HP de l'aptamère. Chaque injection (échantillons et blancs) a été réalisé en duplicate. La puce permet d'étudier en temps réel la formation et la rupture des interactions entre le FVII HP et l'aptamère immobilisé grâce à la résonance plasmonique de surface (RPS). Une liaison sur l'aptamère immobilisé se traduit par une augmentation du signal en unité de résonance (RU) enregistré par l'appareil (Figure 1). Ces analyses sont effectuées avec l'appareil de RPS Biacore T100((GE). La modélisation des interactions enregistrées sont effectuée à l'aide du Logiciel Biaevaluation((GE). A l'aide d'un modèle de liaison allostérique, le Kd de liaison entre l'aptamère et le FVII HP a été estimé à 1.4µM.

Afin de vérifier que le produit qui se lie à l'aptamère immobilisé est bien le FVII, une séquence d'injection comprenant (i) le FVII HP à 200nM et (ii) un anticorps polyclonnal anti-FVII à 100nM (Abcam) a été réalisée sur la même puce. Les conditions d'injection et d'analyse sont identiques à celle décrites précédemment. Si l'aptamère retient effectivement du FVII, l'injection d'anticorps polyclonnal anti-FVII doit se traduire par une augmentation du signal en RU consécutif à la liaison des anticorps sur le FVII lui même lié à l'aptamère. En témoins des anticorps seuls sont injectés. L'augmentation de signal en RU montre bien que l'aptamère reconnaît le FVII (Figure 2).

Cet exemple montre que l'aptamère, une fois immobilisé se lie spécifiquement au FVII HP, avec une affinité significative.

### Exemple 2 : Liaison de l'aptamère (SEQ n°1) avec du FVII de lapin

Du FVII plasmatique de lapin (American Diagnostica) a été dilué dans le même tampon que dans l'exemple 1 à des concentrations de 50, 100 200, 400nM puis injecté dans les mêmes conditions que l'exemple 1 sur la même puce contenant l'aptamère immobilisé.

Les signaux enregistrés en RU, montrés sur la Figure 3 montrent l'absence totale de liaison de l'aptamère pour le FVII de lapin et ce malgré une forte homologie de séquence en acides aminés entre le FVII de lapin et le avec le FVII HP (environ 75% d'homologie de séquence en acides aminés).

Cet exemple montre que l'aptamère se lie spécifiquement au FVII HP mais ne se lie pas au FVII de lapin.

### Exemple 3 : différences d'affinité vis à vis de l'aptamère (SEQ n°1) entre le FVII HP, le FVII transgénique et le FVII de lapin

Des injections successives de FVII HP, de FVII plasmatique de lapin et de FVII transgénique humain ont été réalisées dans des conditions identiques à l'exemple 1 sur une même puce. Les données obtenues (Figure 4) montrent que l'aptamère se lie au FVII HP et au FVII transgénique, avec des affinités différentes mais néanmoins fortes, mais ne se lie pas au FVII de lapin.

## Revendications

1. Procédé pour purifier spécifiquement un facteur de la coagulation, à partir d'une solution biologique provenant d'un animal transgénique non-humain qui contient ledit facteur de la coagulation et qui est susceptible de contenir une protéine homologue audit facteur de la coagulation, le procédé comprenant les étapes de :
a) fournir un support solide comprenant un aptamère d'ADN immobilisé audit support solide *via* un espaceur **caractérisé en ce que** ledit aptamère immobilisé se lie spécifiquement audit facteur de la coagulation mais ne se lie pas à une protéine homologue audit facteur de la coagulation,
b) mettre en contact ledit support solide avec ladite solution biologique, et
c) récupérer le facteur de la coagulation lié audit aptamère.

2. Procédé selon la revendication 1, **caractérisé en ce que** le facteur de la coagulation présente une identité de séquence en acides aminés supérieure à 50 % avec ladite protéine homologue, de préférence supérieure à 60%, de préférence supérieure à 70%, de préférence supérieure à 80%, de préférence supérieure à 90% avec ladite protéine homologue.

3. Procédé selon l'une quelconque des revendications 1 ou 2, **caractérisé en ce que** le facteur de la coagulation est choisi parmi le groupe constitué par le facteur VII (FVII), le facteur VIII (FVIII), le facteur X (FX), le facteur IX (Facteur IX), le facteur XI (FXI), le facteur XII (FXII), le facteur XIII (FXIII), le facteur II (THROMBINE), l'antithrombine III (AT III) le cofacteur II de l'héparine (HCII), la protéine C (PC), la thrombomoduline (TM), la protéine S (PS), le facteur V (FV), le facteur de Willebrand (FvW) ou l'inhibiteur de la voie du facteur tissulaire (TFPI).

4. Procédé selon la revendication 3, **caractérisé en ce que** l'aptamère a une séquence nucléotidique au moins 80 % identique à la SEQ ID n°1.

5. Procédé selon l'une quelconque des revendications 1 à 4, **caractérisé en ce que** ledit support solide est une puce, une colonne de chromatographie, une bille magnétique ou paramagnétique ou une membrane.

6. Procédé selon l'une quelconque des revendications 1 à 5, **caractérisé en ce que** ledit espaceur est une séquence oligonucléotidique non spécifique ou une séquence de type polyéthylène glycol (PEG).

7. Procédé selon l'une quelconque des revendications 1 à 6 **caractérisé en ce que** ladite solution est un fluide corporel,

8. Procédé selon l'une quelconque des revendications précédentes dans lequel la solution biologique est choisie dans le groupe constitué par du lait, un dérivé du lait, du sang ou un dérivé du sang.

9. Procédé selon l'une quelconque des revendications précédentes dans lequel la solution biologique est choisie dans le groupe constitué par un plasma, un cryoprécipité du plasma, un lait clarifié et leurs dérivés.

10. Procédé selon l'une quelconque des revendications précédentes dans lequel la solution biologique est obtenue à partir d'un mammifère transgénique non-humain.

11. Procédé selon la revendication 10, **caractérisé en ce que** la solution biologique est du lait ou un dérivé du lait dudit mammifère transgénique non-humain.

12. Procédé selon l'une quelconque des revendications précédentes dans lequel le facteur de la coagulation est le facteur VII humain.

## Patentansprüche

1. Verfahren zur spezifischen Reinigung eines Koagulationsfaktors aus einer von einem nichthumanen transgenen Tier stammenden biologischen Lösung, die besagten Koagulationsfaktor enthält und die ein zu besagtem Koagulationsfaktor homologes Protein enthalten kann, wobei das Verfahren die Schritte umfasst:
a) Bereitstellen eines festen Trägers, umfassend ein DNA-Aptamer, das an besagten festen Träger *über* einen Spacer immobilisiert ist, **dadurch gekennzeichnet, dass** besagtes immobilisiertes Aptamer an besagten Koagulationsfaktor spezifisch bindet, aber nicht an ein zu besagtem Koagulationsfaktor homologes Protein bindet,
b) Inkontaktbringen besagten festen Trägers mit besagter biologischer Lösung, und
c) Wiedergewinnen des an besagtes Aptamer gebundenen Koagulationsfaktors.

2. Verfahren gemäß Anspruch 1, **dadurch gekennzeichnet, dass** der Koagulationsfaktor eine Aminosäuresequenz-Identität von größer als 50% mit besagtem homologen Protein, vorzugsweise größer als 60%, vorzugsweise größer als 70%, vorzugsweise größer als 80%, vorzugsweise größer als 90% mit besagtem homologen Protein aufweist.

3. Verfahren gemäß einem der Ansprüche 1 oder 2, **dadurch gekennzeichnet, dass** der Koagulationsfaktor aus der Gruppe ausgewählt ist, bestehend aus Faktor VII (FVII), Faktor VIII (FVIII), Faktor X (FX), Faktor IX (Faktor IX), Faktor XI (FXI), Faktor XII (FXII), Faktor XIII (FXIII), Faktor II (THROMBIN), Antithrombin III (AT III), Heparin-Cofaktor II (HCII), Protein C (PC), Thrombomodulin (TM), Protein S (PS), Faktor V (FV), von-Willebrand-Faktor (FvW) oder Gewebefaktorweghemmer (TFPI).

4. Verfahren gemäß Anspruch 3, **dadurch gekennzeichnet, dass** das Aptamer eine Nukleotidsequenz besitzt, die wenigstens zu 80% mit SEQ ID NR: 1 identisch ist.

5. Verfahren gemäß einem der Ansprüche 1 bis 4, **dadurch gekennzeichnet, dass** besagter fester Träger ein Chip, eine Chromatographie-Säule, ein magnetisches oder paramagnetisches Kügelchen oder eine Membran ist.

6. Verfahren gemäß einem der Ansprüche 1 bis 5, **dadurch gekennzeichnet, dass** besagter Spacer eine nicht-spezifische Oligonukleotid-Sequenz oder eine Sequenz vom Typ Polyethylenglycol (PEG) ist.

7. Verfahren gemäß einem der Ansprüche 1 bis 6, **dadurch gekennzeichnet, dass** besagte Lösung eine Körperflüssigkeit ist.

8. Verfahren gemäß einem der vorhergehenden Ansprüche, wobei die biologische Lösung aus der Gruppe ausgewählt ist, bestehend aus Milch, einem Milchderivat, Blut oder einem Blutderivat.

9. Verfahren gemäß einem der vorhergehenden Ansprüche, wobei die biologische Lösung aus der Gruppe ausgewählt ist, bestehend aus einem Plasma, einem Plasma-Kryopräzipitat, einer geklärten Milch und Derivaten davon.

10. Verfahren gemäß einem der vorhergehenden Ansprüche, wobei die biologische Lösung von einem nicht-humanen transgenen Säugetier erhalten wird.

11. Verfahren gemäß Anspruch 10, **dadurch gekennzeichnet, dass** die biologische Lösung Milch oder ein Milchderivat besagten nicht-humanen transgenen Säugetiers ist.

12. Verfahren gemäß einem der vorhergehenden Ansprüche, wobei der Koagulationsfaktor der humane Faktor VII ist.

## Claims

1. A method for specifically purifying a coagulation factor, from a biological solution obtained from a non-human transgenic animal which contains said coagulation factor and which may contain a protein homologous to said coagulation factor, the method comprising the steps of:
a) providing a solid support comprising a DNA aptamer immobilized onto said solid support through a spacer, **characterized in that** said immobilized aptamer specifically binds to said coagulation factor but does not bind to a protein homologous to said coagulation factor,
b) contacting said solid support with said biological solution, and
c) recovering the coagulation factor bound to said aptamer.

2. The method according to claim 1, **characterized in that** the coagulation factor has an amino-acid sequence identity of more than 50% with said homologous protein, preferably of more than 60%, preferably of more than 70%, preferably of more than 80% preferably of more than 90% with the homologous protein.

3. The method according to any one of claims 1 or 2, **characterized in that** said coagulation factor is selected from the group consisting of factor VII (FVII), factor VIII (FVIII), factor X (FX), factor IX (Factor IX), factor XI (FXI), factor XII (FXII), factor XIII (FXIII), factor II (THROMBIN), antithrombin III (AT III), heparin cofactor II (HCII), protein C (PC), thrombomodulin (TM), protein S (PS), factor V (FV), von Willebrand factor (FvW) and tissue factor pathway inhibitor (TFPI).

4. The method according to claim 3, **characterized in that** the aptamer comprises a nucleotide sequence at least 80% identical to SEQ ID n°1.

5. The method according to any one of claims 1 to 4, **characterized in that** said solid support is a chip, a chromatography column, a magnetic or para-magnetic bead or a membrane.

6. The method according to any one of claims 1 to 5, **characterized in that** said spacer is a non-specific oligonucleotide sequence or a sequence of the polyethylene glycol (PEG) type.

7. The method according to any one of claims 1 to 6 **characterized in that** said solution is a body fluid.

8. The method according to any one of preceding claims, wherein the biological solution is selected in the group consisting of milk, a milk-derived product, blood or a blood-derived product.

9. The method according to any one of preceding claims, wherein the biological solution is selected in the group consisting of plasma, plasma cryoprecipitate, clarified milk and derivatives thereof.

10. The method according to any one of preceding claims, wherein the biological solution is obtained from a transgenic non-human mammal.

11. The method of claim 10, **characterized in that** the biological solution is milk or a derivative of milk from a non-human transgenic mammal.

12. The method according to any one of preceding claims, wherein the coagulation factor is human factor VII.
